(19) Europäisches Patentamt
European Patent Office
Office européen des brevets

(11) Publication number: **0 298 064 A2**

(12) # EUROPEAN PATENT APPLICATION

(21) Application number: **88850221.8**

(22) Date of filing: **21.06.88**

(51) Int. Cl.⁴: **A 61 K 49/00**
C 07 K 15/00, G 01 N 33/577,
C 12 P 21/00
//(C12P21/00,C12R1:91)

(30) Priority: **30.06.87 SE 8702711**

(43) Date of publication of application:
**04.01.89 Bulletin 89/01**

(84) Designated Contracting States:
**AT BE CH DE ES FR GB IT LI NL SE**

(71) Applicant: **BIOCARB AB**
**S-223 70 Lund (SE)**

(72) Inventor: **Lundblad, Arne**
**Bytaregatan 7**
**S-222 21 Lund (SE)**

**Lindenberg, Sven**
**Fasanvänget 513**
**DK-2980 Kokkedal (DK)**

(74) Representative: **Henningsson, Gunnar et al**
**Bergling & Sundbergh AB Box 7645**
**S-103 94 Stockholm (SE)**

(54) **Birth control and diagnostic methods.**

(57) A method of controlling uterine implantation of embryos in mammals including man, comprising the step of administering an effective amount of a carbohydrate preparation comprising as an active constituent a structural element having the formula:

EMI ID=21/1 HE=45 WI=95 TI=CHE

wherein:

X is selected from -O-, -S- and -NR-, where R is hydrogen or an acceptable organic residue;

$R_1$ and $R_2$ are the same or different and are selected from H, OH, $NH_2$, NHAc and acceptable organic residues; and

$R_3$ is hydrogen, an organic residue or a carrier;

to a mammal to obtain presence in its uterine luminal environment of such a structural element;

antibody specifically recognizing such structural element;

a diagnostic method for determining mammalian fertility by determining the presence of structural element (I) in uterine cells or uterine secretion, comprising the steps:

a) generating an antibody as defined above;

b) contacting the antibody resulting from step a) with the endometrium of a mammal subject to diagnosis; and

c) determining the degree of interaction between antibody and endometrium so as to evaluate fertility; and

a diagnostic method for determining implantation ability of mammalian blastocysts in vitro, comprising the steps:

a) labelling a structural element (I) as defined above;

b) bringing the labelled element resulting from step a) into contact with a culture of blastocysts to be tested; and

c) recording the degree of interaction between labelled element and blastocysts so as to be able to select useful ones.

EP 0 298 064 A2

## Description

### Birth control and diagnostic methods

The present invention relates quite generally to new birth control techniques, specifically to methods of controlling uterine implantation of embryos in mammals. The invention also involves diagnostic methods for determining mammalian fertility and implantation ability.

BACKGROUND ART

Presently several techniques for anti-conception are used, and a widespread method is constituted by regular administration of hormon containing pills. Such methods have severe drawbacks in that several major side effects may arise as a result of such hormonal treatment. Examples of such side effects are thrombosis, hypertension, artherosclerosis, disturbance of ovulation, bleeding and cancer.

The present invention has for a main purpose to provide a new anti-conception techniques not involving any undesired side effects, such as those mentioned above.

A further object of the invention is to provide new techniques for promoting uterine implantation.

Another object of the invention is to provide a diagnostic method enabling determination of mammalian fertility and of implantation ability of mammalian blastocysts.

Finally, it is an object of the invention to provide antibodies for diagnostic or inhibitory (implantation) use.

Subsequent to mammalian fertilization (including man) the embryo cleaves and within a couple of days it reaches the blastocyst stage. The blastocyst then starts to implant in the uterus. This implantation process includes a) an adhesional phase, b) a nidatory phase and c) the final placentation. In the adhesional phase and the initial nidatory phase the blastocyst surfaces (trophectoderm) interact in a special manner with the uterine tissue (see Enders, A.C. (1976) J.Reprod.Fert.Suppl. 25, 1-15).

Through extensive research and experimentation it has been possible to show for the first time that the implantation process involves a binding system similar to lock and key. The present invention demonstrates that this binding system involves the presence of certain oligosaccharide structures on the uterine luminal surface and corresponding oligosaccharide-binding moieties on the blastocyst surface. Although the invention is not to be limited to any specific theory it is likely that these oligosaccharide-binding moieties are lectins or lectin-related substances.

This basic finding of the present invention can be used in several applications, such as the control or modulation of uterine implantation of embryos in mammals including man, diagnostic methods for determining mammalian fertility and for determining implantation ability of mammalian blastocysts in vitro.

BRIEF SUMMARY OF THE INVENTION

To attain the above and other objects the invention provides for a method of controlling or modulating uterine implantation of embryos in mammals including man, the said method comprising the step of administering an effective amount of a carbohydrate preparation comprising as an active constituent a structural element having the formula:

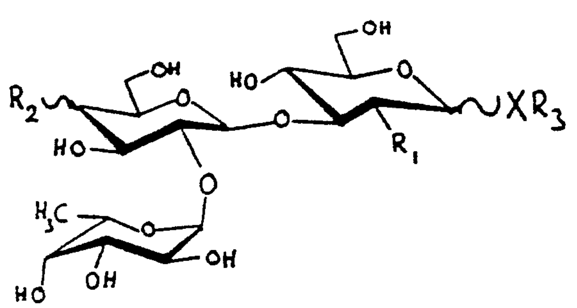

(I)

wherein:

X is selected from -O-, -S- and -NR-, where R is hydrogen or an acceptable organic residue;

$R_1$ and $R_2$ are the same or different and are selected from H, OH, $NH_2$, NHAc and acceptable organic residues; and

$R_3$ is hydrogen, an organic residue or a carrier;

The purpose of such administration is to obtain presence of such a structural element in the uterine luminal environment of the said mammal.

The administration may be of any type as long as the desired presence of the structural element in the uterine luminal environment is obtained. Thus, the administration may be systemic or it may be constituted by a

local application.

The discovery of the lock and key binding system in accordance with the present invention makes it possible to either inhibit uterine implantation or to promote uterine implantation. In the first case the carbohydrate preparation as defined above is brought into contact with the embryo (blastocyst) so as to inhibit implantation of said embryo (blastocyst). In the second aspect of the invention the endometrium or uterine mucous membrane is treated with such a carbohydrate preparation to increase embryotic (blastocyst) adhesion to the endometrium.

The invention also includes within its scope antibodies specifically recognizing the structural element (I) as defined above, and such antibodies are preferably of the monoclonal type. Such antibodies may be used for inhibiting uterine implantation by treating the endometrium with the antibody for the purpose of inhibiting embryotic adhesion to the endometrium.

According to yet another aspect of the invention such antibodies may be used in a diagnostic method for determining mammalian fertility. In such a diagnostic method the presence of structural element I (as defined above) in uterine cells or uterine secretion, is determined by contacting an antibody (as defined above) with the endometrium of a mammal including man subject to diagnosis, and determining the degree of interaction between antibody and endometrium for the purpose of evaluating fertility.

According to still another aspect of the invention there is provided a diagnostic method for determining implantation ability or capacity of mammalian blastocysts in vitro. Such a diagnostic method comprises the steps:

a) labelling a structural element (I) as defined above;

b) bringing the labelled element into contact with a culture of blastocysts to be tested; and

c) recording the degree of interaction between labelled element and blastocysts so as to enable selection of useful blastocysts.

The diagnostic procedure for performing test for infertility thus makes it possible during in vitro fertilization (IVF) and embryo transfer treatment (ET) to select embryos having the receptor for the structural element I. Blastocysts having such receptor having a higher possibility of giving live offsprings than embryos without this receptor. Accordingly, the present invention offers a useful viability test for embryos in an IVF-ET-treatment.

In the structural element I as defined above substituents $R_1$ and $R_2$ can be the same or different and are selected from H, OH, $NH_2$, NHAc, and acceptable organic residues, preferably of low molecular weight, for example less than about 200, and preferably less than about 100. Preferred substituents are selected from H, OH, NHAc, $NH_2$, O-acyl and NHR, wherein R is lower alkyl or lower acyl.

In the present disclosure the term "lower" indicates a maximum of 6 carbon atoms in the substituent.

In formula (I) X is preferably -O- and R(-NR-) is preferably selected from hydrogen, lower alkyl and lower acyl, in particular acetyl.

Substituents $R_1$ and $R_2$ are particularly selected from H, OH, NHAc and OAc. Especially preferred substituents are OH, NHAc and OAc. A particularly preferred combination is a structural element wherein $R_1$ is NHAc and $R_2$ is OH. Although $R_2$ can be either axially or equatorially oriented, axial orientation is preferred.

In regard to substituent $R_3$ of structural element I a much higher degree of freedom in selection is conceivable. Thus, $R_3$ may be hydrogen, an organic residue or a carrier. It is important to note that the expression "carrier" when falling within the definition of $R_3$ always relates to a covalently linked carrier.

$R_3$ may be selected from acyl and carbohydrate residues, and such residues may be constituted by a mono- or oligosaccharide moiety.

Under the definition of $R_3$ as being a covalently linked carrier all types of carriers are conceivable. Thus, the carrier may be a macromolecular one or it may be of the lipid type, particularly in the case where the structural element is being used for promoting uterine implantation by a local application of the carbohydrate preparation according to the invention.

Other examples of $R_3$ are lower alkyl, acetyl.

Under carbohydrate residues there may be mentioned residues comprising galactose and lactose, lactose residues being particularly preferred. When $R_3$ is different from hydrogen $XR_3$ is preferably in the β-configuration.

A particularly preferred structural element has the formula:

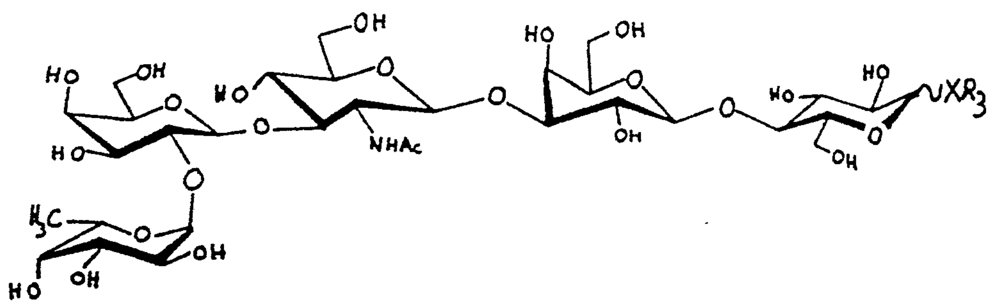 (II)

wherein $R_3$ has the meaning as outlined in the present disclosure.

Further, with regard to the definition of $R_3$, it can be constituted by a macromolecular carrier, optionally covalently linked to the carbohydrate structure via a linking arm. Useful carriers may be synthetic or naturally occurring polypeptides, polysaccharides or other types of polymers, oligomers or particles. For details concerning covalently linked carriers reference is made to US patent 4,137,401 and Svensson, S.B. and Lindberg A.A. (1979) J.Immunol.Meth. 25, 323 and McBroom, C.R., Samanen, C.H., Goldstein, I.J. In: Methods in Enzymology, Vol. 28B, ed. V. Ginsburg, p.212. Academic Press, New York (1972). The disclosures of these are incorporated herein by reference thereto.

The active structural element according to the present invention as defined above can further be formulated together with acceptable carriers or diluents. In this context the expression "carrier" relates to a non-covalently associated material of a conventional nature. The active constituent according to the pre sent invention may be formulated for local application, for example entrapped in a gel. In regard to the application of the present invention for promoting uterine implantation preferred covalently linked carriers are peptides, proteins, oligomeric and polymeric compounds, and lipids.

For a better understanding of the present invention it will now be further illustrated by specific examples of a non-limiting character.

EXAMPLE 1

In vitro implantation - mouse model

Materials and Methods

The following oligosaccharides were obtained from BioCarb Chemicals (S-223 70 Lund, Sweden).

Substance | Substance No:

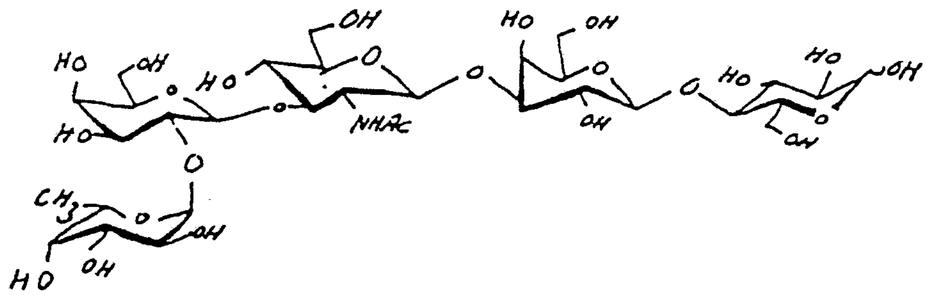

A

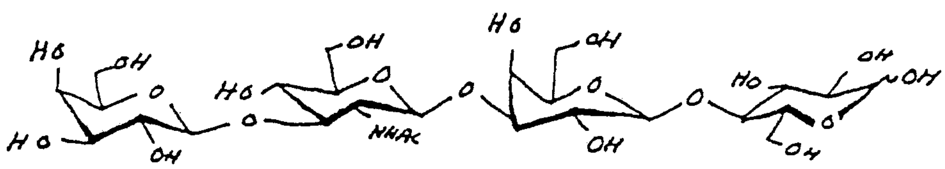

B

C

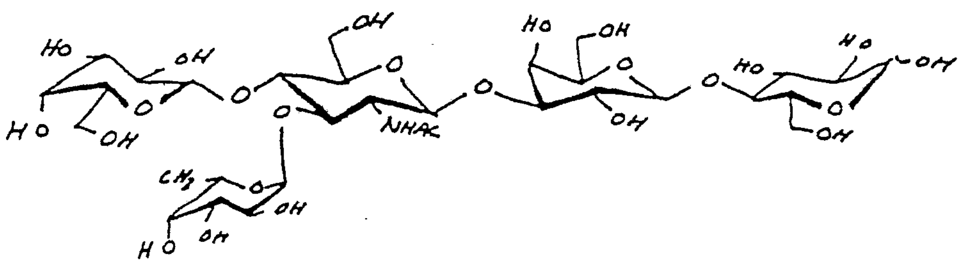

The test system:

In the mouse in vitro implantation system blastocyst implantation in murine endometrial epithelium monolayers was compared in 2 different media: the control medium (i.e. a medium which had supported more than 80% murine blastocyst formation from 2-cell embryos within 72 hours) and test medium (i.e. identical with the control medium except supplemented with 0.1 mM test oligosaccharide).

As control medium we used Earle's minimum essential medium (MEM) (Flow Lab), supplemented with penicillin-G 0.0075 g/l, streptomycin 0.075 g/l, glutamine 1 mM, fetal calf serum 5% and a vegetable protein source 2% (Ultrose-G, LKB, 2216-100). The final osmolality was adjusted to 285 mOsmol/kg and a pH of 7.4.

The mice were F1 hybrid B6D2F1/Bom (from the Laboratory of Animal Breeding and Research Center, Gl.Bomholdt Gard, LTv., 8680 Ry, Denmark). Females aged 26 days and weighing 13-18 g and mature 6 months old males of previously proven fertility were used. They were housed in air-conditioned quarters with pelleted food and water available ad libitum and light between 7.30 a.m. and 7.30 p.m.

The female mice were superovulated with 5 I.U. FSH/LH (Antex, Leo Pharmaceuticals, Denmark) injected intraperitoneally at 9 a.m. day 1. 48 hours later (on day 3), ovulation was induced with 2.5 I.U. HCG. They were caged with males at 1 p.m. and left overnight. Mating was confirmed on the morning of day 4 by the presence of a vaginal plug.

Females were sacrificed at 9 a.m. on day 5. The oviducts were isolated and the 2-cell embryos flushed from the tubes using hand drawn glass pipettes.

Handling the 2-cells embryos outside the $CO_2$ incubator was carried out in the control medium supplemented with a HEPES buffer 20 mM (Flow Lab. 16-884-49).

After the oviducts were collected, the uterus of each mouse was isolated. The uterus was opened longitudinally and scraped with a rubber policeman on the uterine epithelial surface to col lect endometrial cells. These cells were handled in the HEPES buffered control medium as well. After 15 minutes in 300 IU/ml of collagenase type 4 (Gibco) the cells were seeded in the D-valine substituted MEM medium, supplemented

with penicillin, streptomycin, glutamine, ultrose and fetal calf serum in 4-chamber dishes (NUNC A/S, Denmark). This medium supports a selective culture of endometrial epithelial cells with no fibroblastic growth.

The 2-cell mouse embryos were cultured in control medium in 4-chamber dishes (NUNC A/S, DK-4000, Roskilde, Denmark). After culture for 72 hours at 37°C in a humidified atmosphere of 5% $CO_2$ in air, blastocysts were transferred to other 4 chamber dishes with uterine epithelium monolayer cells covering the bottom of each chamber. This was carried out so that pooled blastocysts were always randomly allocated to wells containing the control medium or the test medium.

The co-culture of mouse blastocysts and endometrial monolayer cells were observed daily for adhesion and implantation of the embryos. Implantation of a blastocyst was defined as outgrowth and displacement of endometrial cells by trophoectodermal cells from the embryo.

All tests were controlled morphologically by light and transmission electron microscopy. If there were signs of degeneration in the cultures the media were tested for endotoxins (limulus test) and excluded from the test.

## Results

The results of the test are shown in Table 1.

TABLE 1

| Oligosaccharide Substance | Control impl/total[1] | Test impl/total[1] |
|---|---|---|
| A | 166/175 | 134/228 |
| B | 102/146 | 92/129 |
| C | 138/160 | 110/153 |

[1] impl/total = no. of blastocysts implanting/total no. of blastocysts added to the culture.

## Conclusions

These results demonstrate that oligosaccharide A significantly inhibits the implantation of mouse embryos at an oligosaccharide concentration of 0.1 mM.

The inhibitory effect is not due to toxic agents in the media. The embryos were assessed as viable following light microscopy and transmission electron microscopy. The observed variation in the implantation rate of embryos cultured in the media might be explained by the well known fact that the murine blastocysts are in different developmental stages due to the normal biological variation at this time and that the oligosaccharides are only effective during a limited phase during blastocyst development.

## EXAMPLE 2

### Preparation of monoclonal antibodies against substance no. I and III

#### Antibody 630/7H1

Balb/c-mice were immunised with human granulocytes which are known to contain substance C elements. The spleens from the hyper-immunised mice were harvested and a cell suspension was prepared. After gradient centrifugation (to obtain a pure cell preparation) the cells were fused by means of polyethylene glycol (PEG, average molecular weight 1500D) with established B-myeloma cell lines from Balb/c-mice according to known techniques. After cloning, the hybridoma cell-line 630/7H1 was shown by an extensive screening program to produce antibodies specifically binding structure C. The antibody binding to various structure C containing tissues such as early mouse embryos and primordial germ cells could be inhibited by addition of 3 mM of strucutre C. Other closely related oligosaccharides showed no effect up to a concentration of 5 mM.

The cell-line 630/7H1 was propagated on a large scale, the culture medium supernatants were harvested and the antibodies were purified in a conventional manner.

Antibody 667/9E9

Balb/c-mice were immunised with human seminal plasma which is known to contain the structural elements of structure A. The hybridoma were prepared as described for 630/7H1 and the hybridoma cell-line 667/9E9 was shown to produce antibodies specifically binding structure A. The specificity was carefully checked on ELISA using a glycolipid panel having the oligosaccharide structure A and several structures closely related to structure A.

The cell-line 667/9E9 was propagated on a large scale, the culture medium supernatants were harvested and the antibodies were purified in a conventional manner.

EXAMPLE 3

In vitro test of monoclonal antibodies in mouse implantation model

Materials and methods

Monoclonal antibodies:

See Example 2.

Experimental model:

See Example 1. The final concentration of the antibodies was 50 μg/ml.

Results

The results of the inhibition study is presented in Table 2.

TABLE 2

| Monoclonal antibody | Control impl/total[1] | Test impl/total[1] |
|---|---|---|
| 667/9E9 | 30/39 | 10/53 |
| 630/7H1 | 30/39 | 40/55 |

[1] impl/total = no. of blastocysts implanting/total no. of blastocysts added to the culture.

Conclusions

The data above dmeonstrate that antibodies binding to structure A inhibits the implantation.

EXAMPLE 4

Immunohistochemical studies on human and murine endometrium

Materials and methods

Mice.

We used F1 hybrid B6D2F1/BOM or B6D2F1 (from the Laboratory of Animal Breeding, Research Centre Gl. Bomholtgard, Ltv. 8680, Ry, Denmark) maintained on a 12 hour light/12 hour dark regime, and B6CBF1 (from

MRC Laboratories, Carshalton, UK) maintained on a 14 hour light/10 hour dark regime. Mice were given food and water ad libitum.

Superovulated mice.

Females of the strain B6D2F1/BOM aged 21 days and weighing 13-15 g were superovulated with 5 IU. FSH/LH (Antex, Leo Pharmaceutical, Denmark) injected intraperitoneally at 9 a.m., 48 hours later ovulation was induced with 2.5 I.U. HCG (Physix, Leo Pharmaceutical, Denmark). The female mice were caged with males of the same strain and of proven fertility at 1 p.m. and left overnight. Mating was confirmed on the following morning by the presence of a vaginal plug. The day of observation of the plug was designated to be day 1 of pregnancy.

Naturally mated mice.

Mature virgin female mice 10 to 11 weeks old of the strains B6D2F1 and B6CBF1 were caged with males of their own strain and left overnight. Pregnancy was confirmed as already described.

Monoclonal antibodies:

See example 2.

Preparation of tissue.

Uteri were removed from mice 3 hours after HCG injection on plug day 3, 4, 5 and 6 or from non-pregnant mice. The uterine horns were carefully cut transversely into 4 pieces or when decidual swellings were present, each swelling was isolated separately, and immediately mounted in O.C.T. embedding medium (Raymond Lamb, UK) on cork. The tissue was snap frozen in iso-pentane cooled on dry ice and stored at minus 80° C until required. All tissue blocks were sectioned transversely with respect to the uterine lumen at 10 microns on a cryo-microtome. Sections were air-dried on glass slides and stained either unfixed or after fixation for 10 minutes in acetone.

Staining procedures.

Fixed or unfixed sections were hydrated in Dulbecco's phosphate buffer pH 7.2, containing 0.1% Bovine serum albumin and 0.05% Tween 20. All antibodies and sera were diluted in the same buffer. The sections were incubated for 30 minutes in a 1/20 dilution of normal non immune goat serum (DABCO) to prevent nonspecific binding. Monoclonal antibody supernatants were diluted to either 1/10 or 1/20. The sections were overlayered with 50 µl of the primary antibody and incubated overnight in a humidified atmosphere at 4° C. Following this the sections were rinsed 4 times in the buffer and again incubated for 30 minutes in 1/20 normal goat serum. They were then stained with the second antibody, a fluorescein isothiocyanate (FITC) conjugated goat anti-mouse IgM at a final dilution of 1/80. The sections were finally rinsed in the buffer and mounted in 2.5% DABCO (Boehringer Mannheim, Germany) in 80% glycerol.

Duplicate slides each containing 2 to 4 sections from each tissue block were used for each antibody. The controls used to assess any non specific binding were 1) phosphate buffer and 2) normal goat serum, in place of the primary antibody.

Epi-fluorescence microscopy.

The sections were viewed using a Zeiss Epi-fluorescence microscope with a mercury source for illumination and excitation filter BP 450-490 and suppression filter LP 515 and photographed.

Results

Staining was carried out using more than 2 naturally mated and 2 stimulated mice for each stage investigated, and no difference in the patterns of fluorescence was observed between these two groups. The results from both groups have therefore been considered together. Control sections in all cases showed no fluorescence.

667/9E9:

Non pregnant uterus: 667/9E9 stained luminal aspect of all cells of the uterine epithelium, but no other cell surfaces. Sec retion within the lumen also bound the antibody. The staining pattern was punctate in nature compared to the other antibodies which bound to the uterine epithelium.

Days of pregnancy:

The staining pattern was similar to that of the non pregnant uterus, except that the apical surfaces of the endometrial glands and the secretion within these glands now bound antibody.

Day 4 of pregnancy:

Between day 3 and 4 a change in the pattern of staining on the luminal epithelium was observed. The apical staining was now found to be intermittent with stretches of 4-8 cells binding the antibody. Secreted material in the lumen was stained less intensely than on day 3. All surfaces of the glandular cells as well as the secretion within the glands were now positive.

Day 5 of pregnancy:

The staining was again only on particular patches of luminal cells, but in some cases all surfaces of these cells bound antibody. Luminal secretion was reduced in amount but it still stained with the antibody. Glandular secretion was intensely fluorescent and the surfaces of the cells remained positive.

Day 6 of pregnancy:

At this stage of pregnancy the characteristic punctate distribution of this antibody was still present. The stains were continuously distributed along most of the luminal surface, but in some areas the intermittent pattern remained. The surfaces of the endometrial glands were fluorescent but secretion in both glands and lumen was considerably reduced and little stained.

Inhibition

The staining of the endometrial sections (day 4 and 5) by 667/9E9 could be inhibited upon addition of 1 mM of structure A. Addition of other oligosaccharides such as structures B and C could not affect the staining at even higher concentrations.

Cultured endometrium.

The specific staining by the monoclonal antibody 667/9E9 was also observed for the cultured endometrial monolayers described in Example 1.

Blastocysts

Murine blastocysts were found not to bind antibody 667/9E9.

Conclusions

The studies mentioned above demonstrate the presence of structure A at the luminal surface of the endometrium.

## EXAMPLE 5

### Histochemical study of murine blastocysts

The oligosaccharide "structure A" was conjugated with the fluorescence label FITC using a standard coupling technique. The binding of this labeled oligosaccharide to murine blastocyst was investigated in the present study.

Materials and methods

Blastocysts:

21 days old female F1 hybrid mice were superovulated using 5 IU HMG followed 48 hours later by 2.5 IU LH. At the same day the mice were caged with males of previously proven fertility. During the day after mating the females were checked for the presence of plugs. This day is hereafter designated as day 1. At day 2 2-cell embryos were flushed from the tubes and cultured until the blastocyst stage (day 5).

Staining procedure:

The samples were carefully washed 8 times in a PBS medium containing 4% BSA and 0.2% $NaN_3$. The blastocysts were transferred to a chamber containing the same medium plus 1 mg/ml of the FITC labelled "structure A". The samples were incubated in this solution for 30 minutes at room temperature. After the incubation the samples were carefully washed 8 times. The samples were mounted in microslides (Camlab) in a Dabco antifading medium. The samples were visualized using a Zeiss epifluorescence microscope (see Example 4).

Results

The labelled oligosaccharide was found only to stain the trophectoderm (the blastocyst surface).

Conclusion

The data in Examples 4 and 5 demonstrate the presence of "structure A" at the endometrial surface and a "structure A"-binding moiety on the blastocyst surface.

EXAMPLE 6

In vivo inhibition of implantation in the mouse

In this study the effect of structure A, injected into the uteri of naturally mated and unstimulated mice, was investigated.

Materials and methods

Animals:

10 weeks old NMRI mice were caged with NMRI males of previously proven fertility.

Method:

At plug day 4 (at 1 p.m.) the mice were anaestezed using mebumal. The abdomen was opened through a midline incision and the genital tract was identified. A 6-0 selk sutur was placed around the vaginal top intraperitoneally. 5μl of Earle's balanced salt solution (EBS) supplemented with 5 mmol/l of structure A or unsupplemented were injected into the uterine horns. The test group contained 20 mice who were injected with supplemented media containing structure A, where as the controls (15 mice) were injected with EBS unsupplemented medium.

Results:

| Results: | | Impl/horn |
|---|---|---|
| | Structure A | 1.15 |
| | Controls | 4.2 |

Conclusion:

The studies demonstrate that structure A inhibits implantation in vivo in the mouse.

EXAMPLE 7

Immunohistochemical studies of human endometrium

Materials and methods

Uterine Biopsies.

Uterine biopsies were taken from normally ovulating fertile women, who were admitted to Rigshospitalet (Copenhagen) for sterilization (tube ligation). The samples were taken from the curretage normally performed during this operation. The operations were performed around day 21 in the menstrual cycle of the patients (i.e. the day of implantation).
Monoclonal antibody: 667/9E9, see Example 2.
Preparation of tissue: See Example 4.
Staining procedures: See Example 4.
Epi-fluorescence microscopy: See Example 4.

Results

The fluorescence study showed a staining pattern of the 667/9E9 antibody on the human uterine endometrium. The staining of the antibody showed the same patchy appearance as observed for the murine endometrium.

Conclusion

The present example demonstrates the presence of the same "structure A" oligosaccharides in the endometrium of woman and mouse.

EXAMPLE 8

Histochemical study of human blastocysts

The present study investigates the binding of fluorescence-labelled "structure A" to human blastocysts.

Materials and methods

Human blastocysts:

These blastocysts were "spared embryos" from the In Vitro Fertilization Program at Rigshospitalet in Copenhagen, Denmark and at Sahlgrenska Sjukhuset, Göteborg, Sweden.

Methodology:

See Example 5.

Results

The labelled oligosaccharide was shown to bind to the human blastocysts trophectoderm.

Conclusion

The results demonstrate the presence of "structure A"-binding moieties on the blastocyst surface. Example 7 and 8 show that human endometrium and blastocysts have the same "lock and key" units as the corresponding murine tissues.

**Claims**

1. A method of controlling uterine implantation of embryos in mammals including man, comprising the step of administering an effective amount of a carbohydrate preparation comprising as an active constituent a structural element having the formula:

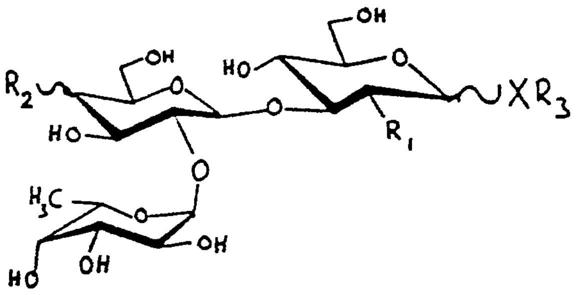

(I)

wherein:

X is selected from -O-, -S- and -NR-, where R is hydrogen or an acceptable organic residue;

$R_1$ and $R_2$ are the same or different and are selected from H, OH, $NH_2$, NHAc and acceptable organic residues; and

$R_3$ is hydrogen, an organic residue or a carrier;

to a mammal to obtain presence in its uterine luminal environment of such a structural element.

2. A method according to claim 1, wherein the administration is constituted by local application.

3. A method according to claim 1 or 2, wherein $R_1$ and $R_2$ are selected from: H, OH, NHAc, $NH_2$, O-acyl and $NHR_4$, where $R_4$ is lower alkyl or lower acyl.

4. A method according to claim 3, wherein $R_1$ and $R_2$ are selected from: H, OH, NHAc, OAc.

5. A method according to claim 4, wherein $R_1$ is NHAc and $R_2$ is OH.

6. A method according to any preceding claim, wherein $R_2$ is axially oriented.

7. A method according to any preceding claim, wherein $R_3$ is selected from H, acyl, and a carbohydrate residue.

8. A method according to any of claims 1 to 6, wherein $R_3$ is a macromolecular carrier.

9. A method according to claim 7, wherein $R_3$ comprises a mono- or oligosaccharide residue.

10. A method according to claim 9, wherein $R_3$ comprises a lactose residue.

11. A method according to claim 1, wherein the structural element is of the formula:

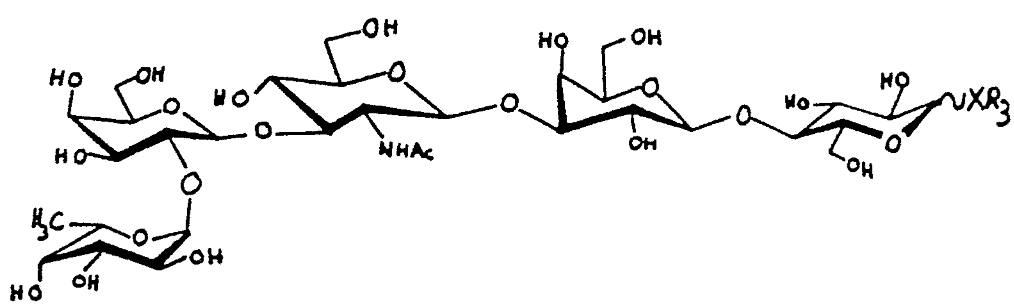

(II)

wherein $R_3$ has the meaning give i claim 1.

12. A method according to claim 11, wherein $XR_3$ is in β-configuration.

13. A method according to any preceding claim for inhibiting uterine implantation by bringing the carbohydrate preparation into contact with the embryo (blastocyst), so as to inhibit implantation of said embryo.

14. A method according to any of claims 1 to 12 for promoting uterine implantation by treating the uterine mucous membrane with said carbohydrate preparation to increase embryonic adhesion thereto.

15. Antibody specifically recognizing the structural element as defined in claim 1.

16. Antibody according to claim 15, which is a monoclonal antibody.

17. A method according to claim 1 for inhibiting uterine implantation by treating the endometrium with an antibody according to claim 15 or 16 to inhibit embryotic blastocyst adhesion thereto.

18. A diagnostic method for determining mammalian fertility by determining the presence of structural element (I) in uterine cells or uterine secretions, comprising the steps:

a) generating an antibody as defined in claim 15 or 16;

b) contacting the antibody resulting from step a) with the endometrium of a mammal subject to diagnosis; and

c) determining the degree of interaction between antibody and endometrium so as to evaluate fertility.

19. A diagnostic method for determining implantation ability of mammalian blastocysts in vitro, comprising the steps:

a) labelling a structural element (I) as defined in any of claims 1 to 12;

b) bringing the labelled element resulting from step a) into contact with a culture of blastocysts to be tested; and

c) recording the degree of interaction between labelled element and blastocysts so as to be able to select useful ones.